Europäisches Patentamt

European Patent Office

Office européen des brevets

(11) Publication number: **0 319 394**
**A1**

## (12) EUROPEAN PATENT APPLICATION

(21) Application number: **88402997.6**

(22) Date of filing: **29.11.88**

(51) Int. Cl.⁴: **A 61 B 1/30**

(30) Priority: **30.11.87 US 126778    29.07.88 US 226059**

(43) Date of publication of application:
**07.06.89 Bulletin 89/23**

(84) Designated Contracting States:
**AT BE CH DE ES FR GB GR IT LI LU NL SE**

(71) Applicant: **Sovak, Milos M. D.**
**La Crescenta P.O. Box 2494**
**Rancho Santa Fe California 92067 (US)**

(72) Inventor: **Sovak, Milos M. D.**
**La Crescenta P.O. Box 2494**
**Rancho Santa Fe California 92067 (US)**

(74) Representative: **Martin, Jean-Jacques et al**
**Cabinet REGIMBEAU 26, Avenue Kléber**
**F-75116 Paris (FR)**

(54) **Hysterography device.**

(57) A hysterography device is provided which avoids the various disadvantages inherent in prior art devices, namely patient discomfort, incomplete seal-off of the cervical canal, complexity, and lack of provision for introduction of catheters or fibrooptic devices into the uterine cavity through a sterile conduit.

According to the invention, the device includes a handle (H) containing one or two conduits ; a cup (C) at one end of the handle (H), designed to fit the cervice of the patient ; means (CV) for applying vacuum within the cup (C); and a cone (CN) within the cup (C), designed to penetrate into the introitus of the cervical canal of the patient.

FIG. 1

**Description**

## HYSTEROGRAPHY DEVICE

### BACKGROUND OF THE INVENTION

This invention pertains to a device providing a sterile conduit for non-invasive entry into the uterine cavity.

A number of conditions require non-invasive entry into the uterine cavity, for both therapeutic and diagnostic purposes. Such access is provided through the canal of the uteral cervix, transvaginally. For diagnostic purposes, contrast media may be injected into the cervical canal and radiography carried out, both to establish the outline of the uterine cavity and/or patency of the Fallopian tubes. Alternatively. ultrasonography can be used following gas insufflation, or a fibrooptic device can be introduced into the uterine cavity for direct inspection. Yet another means of providing access is to introduce catheters into the cervical canal, and input contrast media into the uterine cavity directly.

Recently described in an article entitle "Fallopian Tubes Obstruction: Selective Salpingography and Recanalization," Radiology, May 1987, number 167, pp. 511-514, is the use of a coaxial catheter system with a wire guide for transvaginal recanalization of stenosed Fallopian tubes, also carried out through the cervical canal. This procedure usually is performed in conjunction with diagnostic radiography prior to and following recanalization. Inasmuch as the stenosis usually is a result of inflammatory changes and is one major cause of female infertility, and since surgical transabdominal recanalization of the Fallopian tubes has been shown to meet with only limited success, development of new instrumentation enabling use of non-invasive procedure, such as the radiological approach, is of major importance.

Non-invasive access to the Fallopian tubes is also utilized for the purpose of artificial insemination. Typically, this procedure, which has been shown to be at least twice as effective as in vitro fertilization, involves harvesting the egg and injecting a mixture of sperm and egg into the Fallopian tubes. While this procedure can be carried out under hysteroscopic, radiographic, or ultrasonographic control, the common denominator of all these procedures is access through the cervical canal and insertion of catheters into the tubes. Another possible use of selective tubal catheterization is to inject compositions for the purpose of reversible sterilization.

The internal genital organs must be protected from infectious agents normally or pathologically present in the vaginal environment. A reliably attachable conduit system for safe introduction and manipulation of catheters and other devices, and for the introduction of radiographic contrast media into the uterine cavity and the tubes for the purpose of radiography, does not yet exist. Devices for the introduction of radiographic contrast media for the purpose of uterine/tubal radiography have been described previously, but they have a number of drawbacks, and they do not allow introduction of catheters.

One prior art approach involves grasping the cervix with the tennacula and inserting a rubber cannula into the cervix under tension, with the distinct disadvantages of discomfort to the patient and bleeding from the cervix, as well as incomplete sealing of the cervical canal.

Another prior art device is a large diameter catheter ("Foley catheter") equipped with an occluding balloon into the cervical canal. While sealing with the balloon's inflation is adequate, the Foley catheter does not permit rectification of the uterus by traction, which is often necessary for radiodiagnostic presentation. Neither does the Foley catheter afford insertion of catheters, and thus selective catheterization of the tubes.

Another prior art device, "Malstrom's cannula", employs a cup appositioned to the cervix and held in position by vacuum, forcing a conical rubber cannula into the canal. The seal is accomplished by pressure against the cervical mucosa, but this is not always successful, especially when the entrance to the cervix has been lacerated by previous births. Also the device is complicated, consisting of a large number of parts and requiring considerable manipulation for each resterilization which can be carried out only after thorough dismantling and cleaning. The device does not allow insertion of a catheter.

Another prior art device is the Kidde cannula, devised to seal the canal's orifice with a rubber cone, and to enter the uterine canal with steel tubing. This poses a risk of injury to the uterine cavity, as described, for example, by Winfield, A.C. and Wenz, A.C., "Techniques and Complications of Hysterosalpingography," in Williams and Wilkins (eds.), Diagnostic Imaging of Infertility, 1987, pp. 9-26. Neither this nor any of the prior art devices permit introduction of a coaxial or single catheter for selective tubal catheterization or fibroscope introduction.

### SUMMARY OF THE INVENTION

In accordance with the teachings of this invention, a hysterography device is provided which avoids the various disadvantages inherent in prior art devices, namely patient discomfort, incomplete seal-off of the cervical canal, complexity of the device, and lack of provision for introduction of catheters or fibrooptic devices.

### BRIEF DESCRIPTION OF THE DRAWINGS

Figure 1 is a cross-sectional and end view of one embodiment of this invention designed as a fixer cup hysterograph; and

Figure 2 is a cross-sectional view of a second embodiment of this invention, designed as a movable cup hysterograph.


DETAILED DESCRIPTION

This invention is unique in that a novel device operates in consonance with physiological factors that are dynamic rather than morphological. Thus the circular musculature of the cervix can be stretched by pressure exerted on the walls of the cervical canal. Such "cervical dilation" is undesirable for a seal-off. The novel device utilizes a conduit of increasingly larger diameter which dilates the canal but minimally, while the canal's dilation is to an extent prevented, compressing the circumference of the cervix, which results from the vacuum by forcing the cervix into a conical cup.

As shown in the Figures, one embodiment of a hysterography device constructed in accordance with the teachings of this invention includes, preferably, a conduit CC which is formed along the longitudinal axis of the device, which serves as the axis, rigid or flexible leading handle H, at the top of which is a cup C, designed to fit the cervix. Cup C is cylindrical in its distal portion but, over radius, becomes progressively more conical towards its base CB. The cup is made preferably from a pliable material, to allow its best adaptation to the cervix. Base CB is flat or recessed, and placed over the outlet CO of another conduit CV, through which vacuum generated by a negative pressure device is applied. This results in suction which forces the cervical mucosa against the edge of the cup, and or the cervix into the cup.

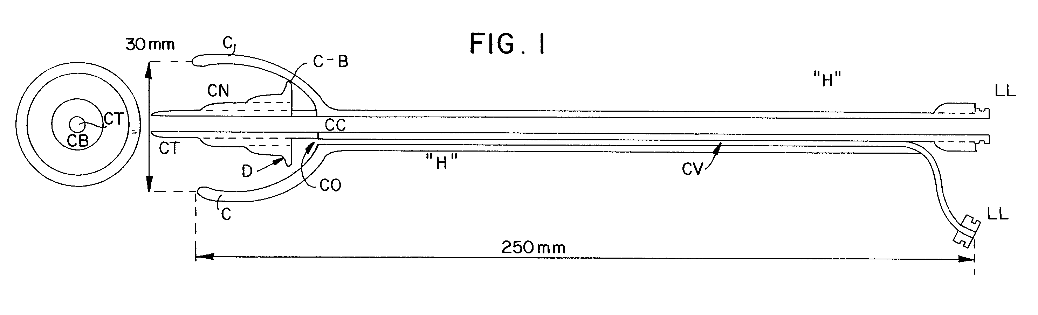

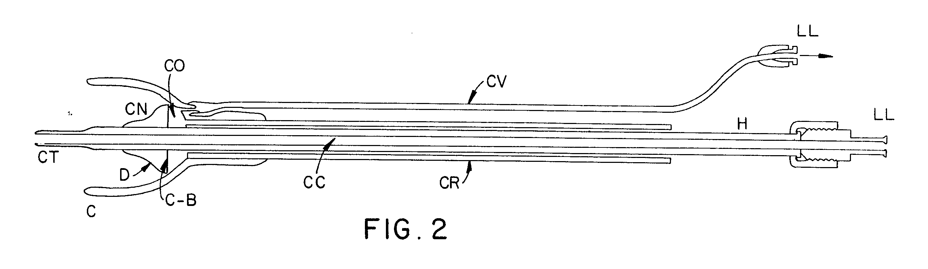

Concentrically and centrally placed into cup C along the axis provided by conduit CC is a conical tube CN, whose proximal, straight part is designed to penetrate into the introitus of the cervical canal. The diameter of the tube CN is then gradually or stepwise increased (from the tip CT to a circular plate D) to provide the primary seal within the canal. As the inner cone of the tube CN is pressed into the cervical canal, its increasingly greater diameter produces the secondary seal-off. Thus, the more vacuum is applied, the more the peripheral mucosa is rolled against and pressed against the inner wall of cup C, thus providing the secondary seal. Finally the perpendicular part D of the cone, while being forced against the frontal mucosa of the cervix, acts as a third sealing surface. The cone can be produced either as one piece, or constructed from several components, with the cup either affixed to the handle H (Figure 1) or to a collar CR surrounding the handle H (Figure 2).

While a version of the hysterograph with the cup affixed to handle H and thus providing a fixed longitudinal relation between the tip of the conduit CC and the cup is useful predominantly for diagnostic purposes, a version employing a movable cup is useful when an anatomical irregularity is encountered for interventional procedures, such as selective or sub-selective catheterization. Such procedures involve manipulations, often resulting in dilation of the cervical canal. When this happens, deeper penetration of the conduit CC into the canal is necessary to provide a seal-off, without which a post-procedural diagnostic radiography could not be carried out. This can be accomplished in a "movable cup" version of the hysterograph by sliding the collar CR, and thus the affixed cup, away from the tip of conduit CC. A seal between the collar CR and handle H is provided by a seal-fit of the diameters; to increase the seal, a silicone or other physiologically inert lubricant can be utilized. Conveniently, cups of several sizes can be interchangeable on the same instrument.

Central conduit CC is contained in the handle H and provides a passage through which a catheter, a coaxial catheter system, or fibrooptic device can be inserted, or through which contrast media can be injected. At the distal end of conduit CC, a standard Luer lock LL is provided to afford attachment of standard syringes and/or catheter collars. A similar lock LL is provided at the distal end of conduit CV, for connection to a vacuum source. The handle H is rigid or flexible and made from a plastic tube containing one or two lumina, one for CC and one for vacuum. Alternatively, the vacuum line can be an independent tube.

If desired, the device conveniently consists of one or several parts, and the cup can be manufactured from suitable see-through plastic materials to allow viewing of the insertion of the conus. The device can be machined and assembled from commercially available components, or molded and produced entirely or in part by standard plastic extrusion methods. Suitable plastic materials include polycarbonate, polyacrylic resins, clear polyethylene, polyvinyl chloride, carbon fiber, fiberglass, and other organic and inorganic polymeric or monomeric compositions.

The devices, intended to fit a wide range of cervical sizes, are easily manufactured and conveniently sterilized and packaged ready for one-time use or repeated use.

The devices can be constructed to have a range of dimensions, as follows:

| Dimension | Approximate Range of Values | Approximate Value in One Embodiment |
|---|---|---|
| Overall length of device (DL) | 220 to 300 mm | 240 mm |
| Outside diameter of cup C (do) | 25 to 40 mm | 33 mm |
| Inside diameter of cup C (di) | 21 to 36 mm | 30 mm |
| Length of cup C | 25 to 35 mm | 27 mm |
| Diameter of conduit CC | 2 to 5 mm | 2,6 mm (at the tip) |
| Length of cone CN | 30 to 40 mm | 35 mm |
| Outside diameter of base CB of cone CN | 15 to 25 mm | 20 mm |
| Outside diameter of tip CT of cone CN | 3 to 6 mm | 4 mm |
| Outside diameter of handle H (may be oval) | 6 to 12 mmm | 7 mm |
| Length of collar CR | 5 to 17 mm | 15 mm |
| Diameter of collar CR | to seal-fit the outer surface of handle H | |

All publications and patent applications are herein incorporated by reference to the same extent as if each individual publication or patent application was specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be practiced within the scope of the appended claims.

**Claims**

1. A hysterography device comprising:
a handle (H) containing one or two conduits;
a cup (C) located at a first end of said handle, said cup designed to fit the cervice of a patient;
means (CV) for applying a vacuum within said cup, made from soft plastic material;
a cone (CN) located within said cup having a nose designed to penetrate into the introitus of the cervical canal of said patient.

2. A hysterography device as in Claim 1 further comprising a vacuum line (CV) having a first end connected to said means for applying a vacuum and having a second end terminating near a second end of said handle.

3. A hysterography device as in Claim 1 wherein said handle (H) further comprises a second end opposite said first end, and wherein said second end includes means (LL) for introducing items into said conduit for application to said patient via said first end of said handle.

4. A hysterography device as in Claim 3 wherein said items are selected from the group of items consisting of catheters, coaxial catheters, fibrooptic devices, and contrast media.

5. A hysterography device as in Claim 1 wherein said cup (C) further comprises an inner wall for pressing upon the cervical mucosa of said patient.

6. A hysterography device as in Claim 5 wherein the pressing of said cervix against said cervical mucosa against said edge of said cup (C) and said inner wall is enhanced by the application of vacuum by said means(CV) for applying a vacuum within said cup.

7. A hysterography device as in Claim 6 wherein said pressing of said cervix against said inner wall and against the frontal plate of the cone (CN) provides a vacuum seal.

8. A hysterography device as in Claim 1 wherein said nose of said cone (CN), together with the conduit, provides a seal within the cervical canal.

9. A hysterography device as in Claim 1 wherein the increasing diameter of said conduit and cone (CN) serves to occlude the cervical canal.

10. A hysterography device as in Claim 1 wherein the cone (CN) is terminated with a circular plate (D) perpendicular to the longitudinal axis, said plate providing additional seal against the frontal portion of the cervix.

11. A hysterography device as in Claims 1 and 6 wherein said cup (C) is movable along the longitudinal axis of said handle (H), by means of a coaxial collar (CR), providing adaptation to the oervix of the cup (C) and variable penetration of the conduit (CC) with the cone (CN) into the cervical canal for the purpose of a seal.

FIG. I

FIG. 2

EP 0 319 394 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-1 445 180 (RICHARD WOLF GMBH) <br> * page 1, lines 20-29; page 2, lines 1-20; fig ure 1 * <br> --- | 1-9 | A 61 B 1/30 |
| A | US-A-2 547 758 (W.B. KEELING) <br> * column 2, lines 6-9; figures 1, 2 * <br> --- | 11 | |
| X | DK-C- 105 355 (AKTIEBOLAGET VACUUMEXTRACTOR) <br> * page 2, left hand column, line 54-page 3, left hand column, line 28 * <br> ----- | 1-10 | |

TECHNICAL FIELDS SEARCHED (Int. Cl.4)

A 61 B 1/00
A 61 B 5/00

The present search report has been drawn up for all claims

| Place of search | Date of completion of the search | Examiner |
|---|---|---|
| BERLIN | 15-02-1989 | WEIHS J.A. |

EPO FORM 1503 03.82 (P0401)